# EUROPÄISCHE PATENTSCHRIFT

(11) **EP 2 688 555 B1**
(45) Veröffentlichungstag und Bekanntmachung des Hinweises auf die Patenterteilung: **01.11.2017**
(21) Anmeldenummer: 12709622.0
(22) Anmeldetag: 15.03.2012
(51) Int. Cl.: A61K 9/16, A61K 31/137

(54) **FESTE AMBROXOLHALTIGE ZUBEREITUNG**
SOLID AMBROXOL-CONTAINING PREPARATION
PRÉPARATION SOLIDE CONTENANT DE L'AMBROXOL

(30) Priorität: 21.03.2011 EP 11159031
(43) Veröffentlichungstag der Anmeldung: 29.01.2014
(73) Patentinhaber: Sanofi-Aventis Deutschland GmbH, 65929 Frankfurt am Main (DE)
(72) Erfinder: BRICKL, Rolf-Stefan, 55216 Ingelheim Am Rhein (DE); KRENKEL, Herrad-Odilia, 55216 Ingelheim Am Rhein (DE)
(74) Vertreter: Catillon, Marie
(86) Internationale Anmeldenummer: PCT/EP2012/054615
(87) Internationale Veröffentlichungsnummer: WO 2012/126813

(56) Entgegenhaltungen:
- EP-A1- 0 208 144
- DATABASE WPI Week 200427 Thomson Scientific, London, GB; AN 2004-286857 XP002657184, -& JP 2004 024686 A (NIPPON SHINYAKU CO LTD) 29. Januar 2004 (2004-01-29)

## Beschreibung

Die vorliegende Erfindung betrifft feste ambroxolhaltige Zubereitungen, wie in den Ansprüchen definiert, erhältlich durch gemeinsame Schmelzextrusion sowie deren Verwendung zur sekretolytische Behandlung bei akuten und chronischen bronchopulmonalen Erkrankungen.

### STAND DER TECHNIK

Ambroxol Hydrochlorid (trans-4-(2-Amino-3,5-dibrombenzylamino)-cyclohexanol Hydrochlorid) ist ein Wirkstoff aus der Gruppe der Expektorantien, der in der Therapie akuter und chronischer Erkrankungen der unteren Atemwege, die mit einer Störung von Schleimbildung und -transport einhergehen, und zur Linderung akuter Halsschmerzen verwendet wird. Ambroxol Hydrochlorid wird dabei in verschiedenen Darreichungsformen verwendet, beispielsweise als Lutschtabletten, Tropfen, Saft, Tabletten, Retardkapseln, Brausetabletten oder Inhalationskonzentrat.

Die kommerziell erhältlichen festen Darreichungsformen von Ambroxol bestehen üblicherweise aus Pellets, die durch Sprüherstarrung hergestellt werden und einen Wirkstoffgehalt von etwa 35 Gew.-% aufweisen.

Nachteil dieses Herstellungsverfahrens ist die starke Abhängigkeit der Wirkstofffreigabe von der Verteilung der Partikelgröße, die wiederum stark von den Sprühparametern abhängt. Dies erfordert einen besonderen Aufwand um die Reproduzierbarkeit der Wirkstofffreigabe zu gewährleisten. Ein weiterer Nachteil des Herstellungsverfahrens ist der niedrige erzielbare Wirkstoffanteil der festen Zubereitung, der üblicherweise aufgrund von Sprühproblemen geringer als 35 % ist.

EP 0 208 144 A1 offenbart Ambroxol-Zubereitungen mit verzögerter Wirkstofffreisetzung zur oralen Anwendung, wobei der Wirkstoff eingebettet in einem niedrigschmelzenden Lipid mit einem Schmelzbereich von 40-90°C vorliegt.

JP 2004 024686 A offenbart Schmelzextrusionsverfahren zur Bereitstellung von Granulaten enthaltend eine Mischung aus 10% Ambroxol HCl in einer wachsartigen Matrix, die u.a. 20% hydriertes Castoröl enthält.

### Aufgabenstellung

Aufgabe der vorliegenden Erfindung war es, feste ambroxolhaltige Zubereitungen bereitzustellen, die durch ein reproduzierbares Verfahren hergestellt werden können und eine gezielte Einstellung der Wirkstofffreisetzung erlauben. Das Herstellverfahren dieser Zubereitung sollte vorzugsweise einfach, robust und für die kontinuierliche Herstellung geeignet sein.

Eine weitere Aufgabe der vorliegenden Erfindung war es feste pharmazeutische Darreichungsformen mit hohem Wirkstoffgehalt bereitzustellen, deren Freigabecharakteristik sich gezielt einstellen lässt, um beispielsweise bei Bedarf ohne weiteres Bioäquivalenz zu den kommerziell erhältlichen festen Darreichungsformen erzielen zu können.

### BESCHREIBUNG DER ERFINDUNG

Diese Aufgaben werden gelöst durch feste ambroxolhaltige Zubereitungen, wie in den Ansprüchen definiert, erhältlich durch Schmelzextrusion einer Mischung bestehend aus a) 30 bis 80 Gew.-% Ambroxol Hydrochlorid, b) 2 bis 68 Gew.-% mindestens eines hydrierten Pflanzenöl, c) 2 bis 68 Gew.-% mindestens eines Gemisches enthaltend Fettsäureester und/oder Hydroxyfettsäureester und d) 0 bis 66 Gew.-% eines oder mehrerer pharmazeutischer Hilfsstoffe, jeweils bezogen auf die Gesamtmenge der Zubereitung.

Speziell betrifft die vorliegende Erfindung ambroxolhaltige Zubereitungen, erhältlich durch Schmelzextrusion einer Mischung bestehend aus a) 30 bis 80 Gew.-% Ambroxol Hydrochlorid, b) 2 bis 68 Gew.-% eines hydrierten Pflanzenöls, wobei das hydrierte Pflanzenöl b) hydriertes Castoröl ist, c) 2 bis 68 Gew.-% mindestens eines Gemisches enthaltend Fettsäureester und/oder Hydroxyfettsäureester, wobei das Gemisch enthaltend Fettsäureester und/oder Hydroxyfettsäureester c) Carnaubawachs ist, und d) 0 bis 66 Gew.-% eines oder mehrerer pharmazeutischer Hilfsstoffe, jeweils bezogen auf die Gesamtmenge der Zubereitung.

Dementsprechend betrifft die vorliegende Erfindung die beschriebenen ambroxolhaltigen Zubereitungen, ein Verfahren zu deren Herstellung, pharmazeutische Darreichungsformen enthaltend die erfindungsgemäßen Zubereitungen sowie die Verwendung der erfindungsgemäßen Zusammensetzungen zur sekretolytische Behandlung bei akuten und chronischen bronchopulmonalen Erkrankungen.

Als Komponente a) wird erfindungsgemäß Ambroxol Hydrochlorid verwendet. Dieses zersetzt sich unter den erfindungsgemäßen Verarbeitungsbedingungen der Schmelzextrusion nicht.

Die Menge der Komponente a) in der erfindungsgemäßen Zubereitung kann je nach gewünschter Freisetzungsgeschwindigkeit in weiten Grenzen variieren. So wird der Ambroxolgehalt üblicherweise im Bereich von 30 bis 80 Gew.-% liegen, bevorzugt im Bereich von 40 bis 80 Gew.-%. Eine spezielle Ausführungsform der vorliegenden Erfindung betrifft Zubereitungen mit einem Ambroxolgehalt im Bereich von 60 bis 80 Gew.-%, vorzugsweise von 70 bis 80 Gew.-% und insbesondere von 75 bis 79 Gew.-%. Überraschenderweise wurde festgestellt, dass mit den erfindungsgemäßen Zubereitungen trotz hohen Ambroxolgehalts ein äquivalentes Freisetzungsprofil zu den kommerziellen Zubereitungen mit geringem Wirkstoffgehalt eingestellt werden kann.

Die Partikelgrösse des in den erfindungsgemässen Zubereitungen verwendeten Ambroxolhydrochlorids ist in weiten Bereichen unkritisch. Die gewählte Partikelgröße kann dabei zur Feineinstellung des gewünschten Freisetzungsprofils dienen. Hierbei gilt grundsätzlich, dass mit zunehmender Partikelgröße die Retardierung zunimmt.

Als Komponenten b) enthalten die erfindungsgemässen Zubereitungen wenigstens ein hydriertes Pflanzenöl, wie in den Ansprüchen definiert. Die erfindungsgemäßen Zubereitungen enthalten Komponenten b) üblicherweise in einer Menge von 2 bis 68 Gew.-%, bevorzugt in einer Menge von 4 bis 30 Gew.-% und besonders bevorzugt in einer Menge von 5 bis 20 Gew.-%, jeweils bezogen auf das Gesamtgewicht der Zubereitung.

Geeignete hydrierte Pflanzenöle b) weisen üblicherweise einen Schmelzpunkt von wenigstens 50°C auf. Der Schmelzpunkt der hydrierten Pflanzenöle bzw. derer Gemische liegt beispielsweise im Bereich von 50 bis 100°C, vorzugsweise 60 bis 95°C, besonders bevorzugt im Bereich von 70 bis 90°C. Hierdurch wird die Extrudierbarkeit der Zubereitung gewährleistet. Als bekannte hydrierte Pflanzenöle b) seien beispielsweise gehärtetes Rizinusöl (hydriertes Castoröl), hydriertes Palmkernöl, hydriertes Palmöl, gehärtete Kokosglyceride, hydriertes Kokosöl oder hydriertes Jojobaöl sowie deren Gemische. In de erfindungsgemässen Zubereitungen wird hydriertes Castoröl als Komponente b) verwendet.

Als Komponenten c) enthalten die erfindungsgemässen Zubereitungen wenigstens ein Gemisch enthaltend Fettsäureester und/oder Hydroxyfettsäureester, wie in den Ansprüchen definiert. Geeignete Gemische c) enthaltend Fettsäureester und/oder Hydroxyfettsäureester weisen üblicherweise einen Schmelzpunkt von wenigstens 45°C auf. Der Schmelzpunkt der Gemische c) enthaltend Fettsäureester und/oder Hydroxyfettsäureester liegt beispielsweise im Bereich von 45 bis 95°C, vorzugsweise 55 bis 90°C, besonders bevorzugt im Bereich von 65 bis 85°C. Hierdurch wird die Extrudierbarkeit der Zubereitung gewährleistet. Als bekannte Gemische c) enthaltend Fettsäureester und/oder Hydroxyfettsäureester seien beispielsweise Carnaubawachs, Candelillawachs, Japanwachs oder Reiskleiewachs sowie deren Gemische genannt. In den erfindungsgemässen Zubereitungen wird Carnaubawachs als Komponente c) verwendet.

Die erfindungsgemäßen Zubereitungen enthalten Komponente c) üblicherweise in einer Menge von 2 bis 68 Gew.-%, bevorzugt in einer Menge von 5 bis 30 Gew.-% und besonders bevorzugt in einer Menge von 5 bis 20 Gew.-%, jeweils bezogen auf das Gesamtgewicht der Zubereitung.

Die Gesamtmenge der Komponenten b) und c) in den erfindungsgemäßen Zubereitungen beträgt dabei üblicherweise von 10 bis 70 Gew.-%, bevorzugt von 12 bis 40 Gew.-%, besonders bevorzugt von 14 bis 30 Gew.-% und ganz besonders bevorzugt von 15 bis 25 Gew.-%, jeweils bezogen auf das Gesamtgewicht der Zubereitung.

Innerhalb der angegebenen Grenzen richtet sich die Menge an Komponente b) relativ zur Menge an Komponente c) bevorzugt danach, welches Freisetzungsprofil gewünscht ist. Üblicherweise liegt das Mengenverhältnis von b) zu c) dabei in einem Bereich von 10 : 1 bis 1 : 10, bevorzugt im Bereich von 5 : 1 bis 1 : 5.

Die Partikelgröße der in den erfindungsgemäßen Zubereitungen eingesetzten Komponenten b) und c) ist in weiten Bereichen unkritisch.

Als Komponenten d) können die erfindungsgemässen Zubereitungen übliche pharmazeutische Hilfsstoffe wie Sprengmittel, Füllstoffe, Schmiermittel, Trennmittel, Fliessregulierungsmittel, Weichmacher und Farbstoffe in Mengen bis zu etwa 66 Gew.-%, bevorzugt von, 0 bis 36 Gew.-%, besonders bevorzugt von 0 bis 5 Gew.-% und ganz besonders bevorzugt von 0 bis 3 Gew.-% enthalten. Diese und die im Folgenden angegebenen Mengen beziehen sich jeweils auf das Gesamtgewicht der Zubereitung.

Als geeignete Sprengmittel seien beispielsweise Crospovidon, Croscarmellose, Natriumalginat, Natriumstärkeglykolat, Carboxymethylnatriumstärkeglykolat, getrocknete Maisstärke und Gemische davon genannt, wobei die Menge an Sprengmittel, sofern verwendet, üblicherweise im Bereich von 0,01 bis 50 Gew.-%, vorzugsweise von 0,05 bis 20 Gew.-% und besonders bevorzugt im Bereich von 0,1 bis 10 Gew.-% liegt.

Als geeignet Füllstoffe seien beispielsweise die Oxide von Magnesium, Aluminium, Silicium und Titan sowie Laktose, Mannit, Sorbit, Xylit, Pentaerythrit und seine Derivate genannt, wobei die Menge an Füllstoff, sofern verwendet, üblicherweise im Bereich von 0,01 bis 5, vorzugsweise von 0,05 bis 2 Gew.-% und besonders bevorzugt im Bereich von 0,1 bis 1 Gew.-% liegt.

Als geeignete Fliessregulierungsmittel seien beispielsweise Wachse wie, Lecithine, Mono-, Di- und Triglyceride langkettiger Fettsäuren, wie C₁₂-, C₁₄-, C₁₆- und C₁₈-Fettsäuren, genannt, wobei die Menge, sofern verwendet, üblicherweise im Bereich von 0,01 bis 5, vorzugsweise 0,05 bis 2 Gew.-% und besonders bevorzugt im Bereich von 0,1 bis 1 Gew.-% liegt.

Als geeignete Weichmacher seien beispielsweise neben niedermolekularen Polyalkylenoxiden wie Polyethylenglykol, Polypropylenglykol und Polyethylenpropylenglykol auch mehrwertige Alkohole wie Propylenglykol, Glycerin, Pentaerythrit und Sorbit sowie Natriumdiethylsulfosuccinat, Mono-, Di- und Triacetat des Glycerin und Polyethylenglykolstearinsäureester genannt, wobei die Menge an Weichmacher, sofern verwendet, üblicherweise im Bereich von 0,01 bis 5, vorzugsweise 0,05 bis 2 Gew.-% und besonders bevorzugt im Bereich von 0,1 bis 1 Gew.-% liegt.

Als geeignete Schmiermittel seien beispielsweise langkettige Alkohole, wie Stearylalkohol, Stearate von Aluminium oder Calcium sowie Talkum und Silikone genannt, wobei die Menge an Schmiermittel, sofern verwendet, üblicherweise im Bereich von 0,01 bis 50 Gew.-%, vorzugsweise von 0,05 bis 20 Gew.-% und besonders bevorzugt im Bereich von 0,1 bis 10 Gew.-% liegt.

Ein weiterer Gegenstand der Erfindung betrifft ein Verfahren zur Herstellung der erfindungsgemäßen Zubereitungen, wobei eine Mischung aus a) Ambroxol Hydrochlorid b) dem hydrierten Pflanzenöl, c) dem Gemisch enthaltend Fettsäureester und/oder Hydroxyfettsäureester und d) gegebenenfalls den Hilfsstoffen bereitgestellt und durch Schmelzextrusion weiterverarbeitet wird.

Das Extrudieren wirkstoffhaltiger Schmelzen wird beispielsweise in EP 0809487, EP 0809488, EP 0854707 oder EP 0998919 beschrieben.

Um die erfindungsgemässen Zubereitungen herzustellen, kann Ambroxolhydrochlorid in Form einer physikalischen Mischung mit den übrigen Komponenten b), c) und d) extrudiert werden, wobei das Mischen der Komponenten a) bis d) vor und/oder während des Extrusionsvorgangs erfolgen kann. Üblicherweise wird man die voneinander getrennten Komponenten a) bis d) gleichzeitig in den Extruder einbringen und während des Extrusionsvorgangs mischen.

Im Übrigen erfolgt die Extrusion aller Komponenten in an sich bekannter Weise in Extrudern, vorzugsweise in Ein- oder Doppelschneckenextrudern in einem Temperaturbereich zwischen 40 und 80°C, bevorzugt 60 bis 75°C. Die Formgebung der ambroxolhaltigen Schmelze zu den erfindungsgemässen Zubereitungen kann beispielsweise durch Düsenplatten mit entsprechenden Bohrungen erfolgen. Die Zerkleinerung des Extrudates in volumengleiche Stücke kann entweder mit rotierenden Messern direkt an der Düsenplatte erfolgen oder die extrudierten Stränge werden nach dem Erstarren in geeigneten Mühlen (z.B. Comill mit Raspelsieben) gemahlen oder in Sphäronizern gebrochen. Die Extrudatstücke können direkt weiterverwendet werden oder sie werden in Sphäronizern bei Temperaturen zwischen 40 und 80 °C, vorzugsweise bei 50 bis 65 °C noch gerundet, um das Fließverhalten zu verbessern.

Die so erhaltenen Extrudate können entweder unmittelbar in loser Form, d.h. als Pellets oder Granulat, mit Dosiersystemen verabreicht oder in Kapseln abgefüllt werden oder nach Mischen mit Tablettierhilfsstoffen in üblichen Tablettiermaschinen zu Tabletten verpresst werden.

Die erfindungsgemässen Zubereitungen sowie die daraus hergestellten Darreichungsformen sind pharmazeutische Darreichungsformen mit verzögerter Wirkstofffreisetzung.

Gewünschtenfalls kann die erfindungsgemäße feste Zubereitung oder pharmazeutische Darreichungsform mit einem üblichen Überzug zur Verbesserung des Aussehens und/oder des Geschmacks oder zur zusätzlichen Verzögerung der Wirkstofffreigabe versehen werden. Die vorliegende Erfindung ermöglicht auf einfache Weise eine gezielte Einstellung des Wirkstofffreisetzungsprofils der erfindungsgemässen pharmazeutische Darreichungsform, vor allem bei der Herstellung von pharmazeutischen Darreichungsformen mit verzögerter Wirkstofffreisetzung.

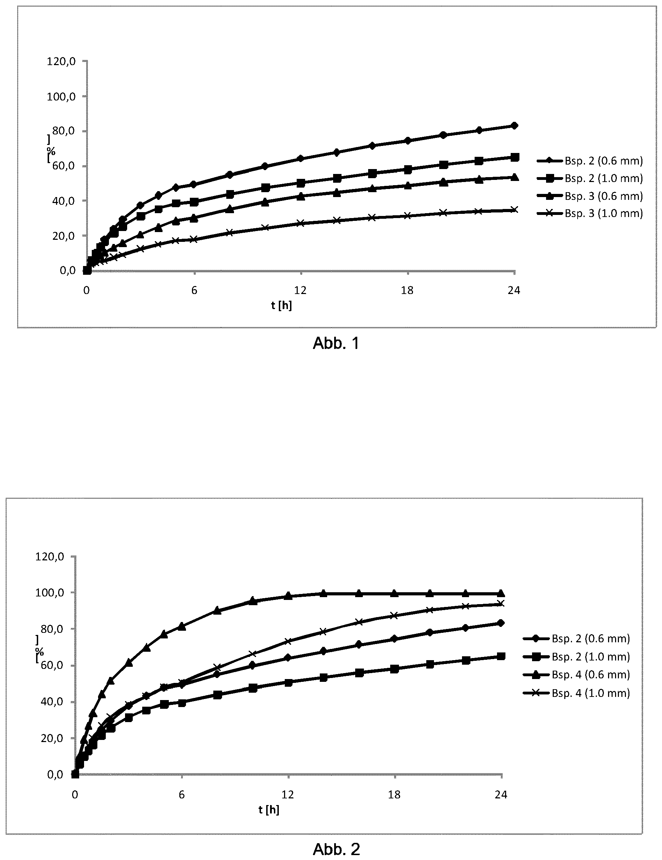

Abbildung 1 zeigt den Einfluss des Anteils an Carnaubawachs auf die Wirkstofffreisetzung sowie den Einfluss der Partikelgröße des Extrudats. Aufgetragen ist die Wirkstofffreisetzung in [%] über die Zeit in [h]. Der Partikeldurchmesser des verwendeten Extrudats der einzelnen Messreihen ist jeweils mit "0.6 mm" bzw. "1.0 mm" angegeben.

Abbildung 2 zeigt den Einfluss eines Sprengmittels auf die Wirkstofffreisetzung sowie den Einfluss der Partikelgröße des Extrudats. Aufgetragen ist die Wirkstofffreisetzung in [%] über die Zeit in [h]. Der Partikeldurchmesser des verwendeten Extrudats der einzelnen Messreihen ist jeweils mit "0.6 mm" bzw. "1.0 mm" angegeben.

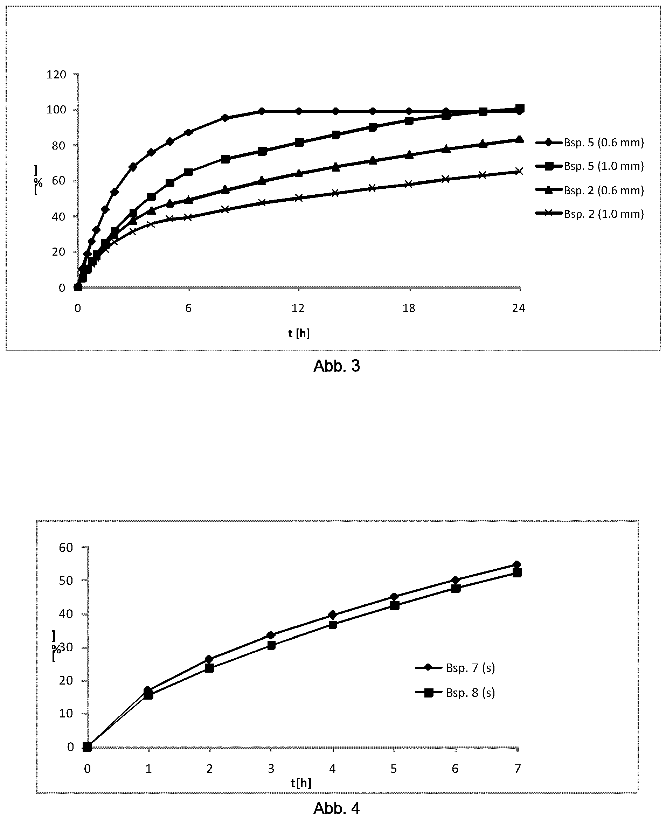

Abbildung 3 zeigt den Einfluss der Partikelgröße von Ambroxolhydrochlorid auf die Wirkstofffreisetzung sowie den Einfluss der Partikelgröße des Extrudats. Aufgetragen ist die Wirkstofffreisetzung in [%] über die Zeit in [h]. Der Partikeldurchmesser des verwendeten Extrudats der einzelnen Messreihen ist jeweils mit "0.6 mm" bzw. "1.0 mm" angegeben.

Abbildung 4 zeigt den Einfluss der Anteile an hydriertem Castoröl und Carnaubawachs auf die Wirkstofffreisetzung. Aufgetragen ist die Wirkstofffreisetzung in [%] über die Zeit in [h]. Die Form (Spheronisierung) des jeweils verwendeten Extrudats ist durch (s) für Stäbchen und (p) für Pellet gekennzeichnet.

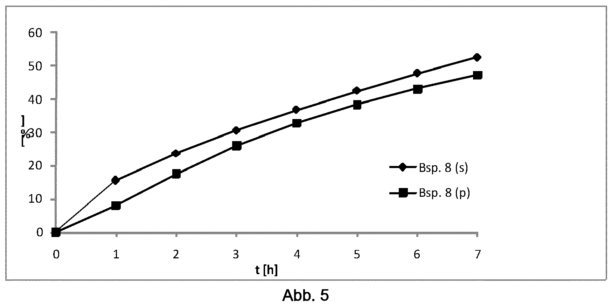

Abbildung 5 zeigt den Einfluss der Spheronisierung auf die Wirkstofffreisetzung. Aufgetragen ist die Wirkstofffreisetzung in [%] über die Zeit in [h]. Die Form (Spheronisierung) des jeweils verwendeten Extrudats ist durch (s) für Stäbchen und (p) für Pellets gekennzeichnet.

Im Folgenden wird die Erfindung anhand nicht einschränkender Beispiele näher erläutert.

### AUSFÜHRUNGSBEISPIEL

### Beispiele 1 bis 6:

**Tabelle 1. Zusammensetzung der Beispiele 1 bis 6**

| Bsp. | Ambroxolhydrochlorid [mg] | hydriertes Castoröl [mg] | Carnaubawachs [mg] | Stearylalkohol [mg] | Kollidon CL [Gew.-%] | Ambroxolhydrochlorid [Gew.-%] |
|---|---|---|---|---|---|---|
| 1 | 30 | 6 | 2 | - | - | 79 |
| 2 | 60 | 12 | 4 | 4 | - | 75 |
| 3 | 60 | 4 | 12 | 4 | - | 75 |
| 4 | 60 | 12 | 4 | 4 | 4 | 71 |
| 5 | 60 | 12 | 4 | 4 | - | 75 |
| 6 | 60 | 12 | 4 | 4 | - | 75 |

Die in der Tabelle 1 angegebenen Mengen an Ambroxolhydrochlorid und den Komponenten b), speziell hydriertes Castoröl mit einem nominalen Schmelzpunkt von 80 °C, c), speziell Carnaubawachs und d), speziell Stearylalkohol und Kollidon CL, wurden gemischt, in einen gleichläufigen 9 mm Doppelschnecken-Extruder (Typ Fa Three-Tec, Schweiz) eingebracht und bei einer Barrel-Temperatur von 78°C extrudiert. Als Düsendurchmesser wurden 0.6 mm und 1.0 mm eingesetzt Die über die Extruderdüsenleiste austretenden Schmelzstränge wurden durch eine Mühle mit Raspelsieb pelletiert, zerkleinert und in Hartgelatinekapseln mit 75 mg Wirkstoffgehalt abgefüllt. Für Beispiele 1 und 4 wurde strahlgemahlener Wirkstoff mit besonders kleiner Partikelgröße, für Beispiele 2, 3, 5 und 6 wurde etwas gröberer stiftgemahlener Wirkstoff eingesetzt.

Die Wirkstofffreisetzung wurde in einer Sotax Dissolution Anlage Typ AT7 mittels Paddlemethode mit 100 Upm in Phosphatpuffer pH 7.5 bei 37 °C über 24 Stunden gemessen. Diese in-vitro-Methode dient zur Bestimmung der Lösungsrate von wirkstoffhaltigen Zubereitungen.

Die Ergebnisse dieses Tests sind in den Abbildungen 1 bis 3 dargestellt.

Abb. 1 zeigt, dass eine Erhöhung des Carnaubawachsanteils die Retardierung deutlich erhöht, ebenso wie eine Erhöhung des Extrudatdurchmessers.

Abb. 2 zeigt, dass der Zusatz eines Sprengmittels die Retardierung deutlich vermindert, ebenso wie eine Erhöhung des Extrudatdurchmessers.

Abb. 3 zeigt, dass eine geringere Partikelgröße des Ambroxolhydrochlorids die Retardierung deutlich vermindert, ebenso wie eine Erhöhung des Extrudatdurchmessers.

### Beispiele 7 bis 10

**Tabelle 2: Zusammensetzungen der Beispiele 7 - 10**

| Bsp. | Ambroxolhydrochlorid [mg] | hydriertes Castoröl [mg] | Carnaubawachs [mg] | Ambroxolhydrochlorid [Gew.-%] |
|---|---|---|---|---|
| 7 | 150 | 30 | 10 | 78.9 |
| 8 | 150 | 25 | 15 | 78.9 |
| 9 | 150 | 20 | 20 | 78.9 |
| 10 | 150 | 10 | 30 | 78.9 |

Die in der Tabelle 2 angegebenen Mengen an strahlgemahlenen Ambroxolhydrochlorid und der Komponente b), speziell hydriertes Castoröl mit einem nominalen Schmelzpunkt von 80 °C, und der Komponente c), speziell Carnaubawachs, wurden gemischt, in einen gleichläufigen 34 mm Doppelschnecken-Extruder (Typ Fa Werner & Pfleiderer) eingebracht und bei einer Material-Temperatur von 70°C extrudiert. Als Düsendurchmesser wurden 0.7 mm bzw. 0.8 mm eingesetzt. Die über die Extruderdüsenleiste austretenden Schmelzstränge wurden in einem Spheronizer bei 36 °C und 1000 Upm 5 Minuten lang zu Stäbchen gebrochen und anschließend bei 60 °C und 100 Upm 20 Minuten lang zu runden Pellets spheronisiert. Dann wurden die Stäbchen bzw. Pellets in Hartgelatinekapseln mit 75 mg Wirkstoffgehalt abgefüllt.

Für Die Wirkstofffreisetzung wurde in einer Sotax Dissolution Anlage Typ AT7 mittels Paddlemethode mit 100 Upm in Phosphatpuffer pH 6.8 bei 37 °C über 7 Stunden gemessen. Diese in-vitro-Methode dient zur Bestimmung der Lösungsrate von wirkstoffhaltigen Zubereitungen.

Die Ergebnisse dieses Tests sind in den Abbildungen 4 und 5 dargestellt.

Abbildung 4 zeigt, dass durch die Variation des Verhältnisses von b), speziell hydriertes Castoröl, und c), speziell Carnaubawachs, eine Feinabstimmung der Wirkstofffreisetzung ohne weiteres möglich ist. Die hierfür verwendeten Zusammensetzungen gemäß Beispielen 7 und 8 lagen jeweils in Stäbchenform (s) vor.

Abbildung 5 zeigt, dass auch durch die Variation der Spheronisierung eine Feinabstimmung der Wirkstofffreisetzung ohne weiteres möglich ist. Die hierfür verwendeten Zusammensetzungen gemäß Beispiel 8 lagen sowohl in Stäbchenform (s) als auch in Pelletform (p) vor.

Diese Beispiele zeigen, dass die Freigabecharakteristik (Wirkstofffreisetzung) über einen sehr weiten Bereich gut steuerbar ist, d.h. es kann nahezu jedes gewünschte Retardierungsprofil eingestellt werden.

## Patentansprüche

1. Feste ambroxolhaltige Zubereitungen, erhältlich durch Schmelzextrusion einer Mischung bestehend aus a) 30 bis 80 Gew.-% Ambroxol Hydrochlorid, b) 2 bis 68 Gew.-% eines hydrierten Pflanzenöls, wobei das hydrierte Pflanzenöl b) hydriertes Castoröl ist, c) 2 bis 68 Gew.-% mindestens eines Gemisches enthaltend Fettsäureester und/oder Hydroxyfettsäureester, wobei das Gemisch enthaltend Fettsäureester und/oder Hydroxyfettsäureester c) Carnaubawachs ist, und d) 0 bis 66 Gew.-% eines oder mehrerer pharmazeutischer Hilfsstoffe, jeweils bezogen auf die Gesamtmenge der Zubereitung.

2. Zubereitungen nach Anspruch 1, wobei das hydrierte Pflanzenöl b) einen Schmelzpunkt von wenigstens 50°C aufweist.

3. Zubereitungen nach einem der vorhergehenden Ansprüche, wobei das Gemisch enthaltend Fettsäureester und/oder Hydroxyfettsäureester c) einen Schmelzpunkt von wenigstens 50 °C aufweist.

4. Verfahren zur Herstellung einer Zubereitungen gemäß einem der vorhergehenden Ansprüche, wobei eine Mischung aus a) Ambroxol Hydrochlorid b) dem hydrierten Pflanzenöl, c) dem Gemisch enthaltend Fettsäureester und/oder Hydroxyfettsäureester und d) gegebenenfalls den Hilfsstoffen bereitgestellt wird und durch Schmelzextrusion weiterverarbeitet wird.

5. Pharmazeutische Darreichungsform enthaltend eine Zubereitungen gemäß einem der Ansprüche 1 bis 3.

6. Darreichungsform gemäß Anspruch 5 zur Verwendung zur sekretolytische Behandlung bei akuten und chronischen bronchopulmonalen Erkrankungen.

## Claims

1. Solid ambroxol-containing preparations obtainable by melt extrusion of a mixture consisting of a) 30 to 80% by weight ambroxol hydrochloride, b) 2 to 68% by weight of a hydrogenated vegetable oil, wherein the hydrogenated vegetable oil b) is hydrogenated castor oil, c) 2 to 68% by weight of at least one mixture comprising fatty acid esters and/or hydroxyfatty acid esters, wherein the mixture comprising fatty acid esters and/or hydroxyfatty acid esters c) is carnauba wax, and d) 0 to 66% by weight of one or more pharmaceutical auxiliaries, based in each case on the total amount of the preparation.

2. Preparations according to Claim 1, wherein the hydrogenated vegetable oil b) has a melting point of at least 50°C.

3. Preparations according to either of the preceding claims, wherein the mixture comprising fatty acid esters and/or hydroxyfatty acid esters c) has a melting point of at least 50°C.

4. Method for preparing a preparation according to any of the preceding claims, wherein a mixture is provided of a) ambroxol hydrochloride, b) the hydrogenated vegetable oil, c) the mixture comprising fatty acid esters and/or hydroxyfatty acid esters and d) optionally the auxiliaries and the mixture is processed further by melt extrusion.

5. Pharmaceutical dosage form comprising a preparation according to any of Claims 1 to 3.

6. Dosage form according to Claim 5 for use for secretolytic therapy in acute and chronic bronchopulmonary disorders.

## Revendications

1. Préparations solides contenant de l'ambroxol, pouvant être obtenues par extrusion à l'état fondu d'un mélange constitué par a) 30 à 80 % en poids de chlorhydrate d'ambroxol, b) 2 à 68 % en poids d'une huile végétale hydrogénée, l'huile végétale hydrogénée b) étant de l'huile de ricin hydrogénée, c) 2 à 68 % en poids d'au moins un mélange contenant des esters d'acides gras et/ou des esters d'hydroxy-acides gras, le mélange contenant des esters d'acides gras et/ou des esters d'hydroxy-acides gras c) étant de la cire de carnauba, et d) 0 à 66 % en poids d'un ou de plusieurs adjuvants pharmaceutiques, à chaque fois par rapport à la quantité totale de la préparation.

2. Préparations selon la revendication 1, dans lesquelles l'huile végétale hydrogénée b) présente un point de fusion d'au moins 50 °C.

3. Préparations selon l'une quelconque des revendications précédentes, dans lesquelles le mélange contenant des esters d'acides gras et/ou des esters d'hydroxy-acides gras c) présente un point de fusion d'au moins 50 °C.

4. Procédé de fabrication d'une préparation selon l'une quelconque des revendications précédentes, dans lequel un mélange de a) le chlorhydrate d'ambroxol, b) l'huile végétale hydrogénée, c) le mélange contenant des esters d'acides gras et/ou des esters d'hydroxy-acides gras et d) éventuellement les adjuvants est préparé et transformé par extrusion à l'état fondu.

5. Forme médicamenteuse pharmaceutique contenant une préparation selon l'une quelconque des revendications 1 à 3.

6. Forme médicamenteuse selon la revendication 5, destinée à une utilisation pour le traitement sécrétolytique dans le cadre de maladies broncho-pulmonaires aiguës et chroniques.
